Europäisches Patentamt

European Patent Office

Office européen des brevets

(11) Veröffentlichungsnummer: 0 214 543
A2

# (12) EUROPÄISCHE PATENTANMELDUNG

(21) Anmeldenummer: 86111747.1

(22) Anmeldetag: 25.08.86

(51) Int. Cl.4: C07C 143/60

(30) Priorität: 07.09.85 DE 3531923

(43) Veröffentlichungstag der Anmeldung:
18.03.87 Patentblatt 87/12

(84) Benannte Vertragsstaaten:
CH DE FR GB IT LI

(71) Anmelder: BAYER AG
Konzernverwaltung RP Patentabteilung
D-5090 Leverkusen 1 Bayerwerk(DE)

(72) Erfinder: Behre, Horst, Dr.
Zur alten Linde 12
D-5068 Odenthal(DE)
Erfinder: Blank, Heinz Ulrich, Dr.
Am Geusfelde 35
D-5068 Odenthal(DE)
Erfinder: Marzolph, Gerhard, Dr.
Semmelweisstrasse 87a
D-5000 Koeln 80(DE)
Erfinder: Streicher, Willi, Dr.
Andreas-Gryphius-Strasse 7
D-5000 Koeln 80(DE)

(54) Verfahren zur Herstellung von 1-Aminoaphthalin-2,4,7-trisulfonsäure und 1-Aminonaphthalin-7-sulfonsäure.

(57) Neues Verfahren zur Herstellung von 1-Amino-naphthalin — 2,4,7-trisulfonsäure und gegebenenfalls Clevesäure-1,7, gemäß dem daß man 1-Nitronaph-thalin in an sich bekannter Weise nach Piria mit Hydrogensulfiten umsetzt, das bei dieser Piria-Reaktion anfallende Sulfaminat-Gemisch mit Schwefeltrioxid in Schwefelsäure zu 1-Aminonaphthalin-2,4,7-tri-sulfonsäure sulfoniert und diese gegebenenfalls durch Erhitzen in wäßriger Schwefelsäure zu Clevesäure-1,7 hydrolysiert.

EP 0 214 543 A2

## Verfahren zur Herstellung von 1-Aminonaphthalin-2,4,7-trisulfonsäure und 1-Aminonaphthalin-7-sulfonsäure

Die Erfindung betrifft ein neues Verfahren zur Herstellung von 1-Aminonaphthalin-2,4,7-trisulfonsäure und 1-Aminonaphthalin-7-sulfonsäure (Clevesäure-1,7) aus 1-Nitronaphthalin.

Clevesäure-1,7 ist ein wichtiges Zwischenprodukt für die Herstellung von Farbstoffen - (siehe Ullmanns Enzyklopädie der Technischen Chemie, 4. Auflage, 1979, Band XVII, Seiten 109-110).

Bislang wird Clevesäure-1,7 in einem vielstufigen Verfahren aus Naphthalin über die Naphthalin-β-sulfonsäure, die zu einem Gemisch aus 5-Nitro- und 8-Nitronaphthalin-2-sulfonsäure nitriert wird, hergestellt. Das Gemisch der beiden isomeren Nitronaphthalinsulfonsäuren wird in die beiden Isomere aufgetrennt und die beiden Isomere zu Clevesäure-1,7 bzw. Clevesäure-1,6 reduziert. Beide Clevesäuren fallen in einer Ausbeute von je 34 % der Theorie an (siehe Winnacker-Küchler, Chemische Technologie, 2. Auflage, 1959, Band 3: Organische Technologie I, Seiten 868-869). Die Auftrennung in die einzelnen Isomere kann jedoch auch erst auf der Stufe der Aminonaphthalinsulfonsäuren vorgenommen werden (siehe Winnacker-Küchler, Chemische Technologie, 4. Auflage, 1982, Band 6: Organische Technologie II, Seite 264). Nachteilig an diesen beiden Verfahren ist die niedrige Ausbeute an Clevesäure-1,7 und der Zwangsanfall an Clevesäure-1,6.

Es hat daher nicht an Versuchen gefehlt, Verfahren aufzufinden, die Clevesäure-1,7 in besseren Ausbeuten liefern und ohne den Zwangsanfall an Clevesäure-1,6. So wird in der DE-OS 25 35 377 die Herstellung von Clevesäure-1,7 durch Ammonolyse und Desulfonierung von 1-Chlornapthalin-4,7-disulfonsäure beschrieben. Dieses Verfahren hat jedoch den Nachteil, daß zur Herstellung der 1-Chlornaphthalin-4,7-disulfonsäure reines 1-Chlornaphthalin benötigt wird, dieses aber technisch nicht preiswert zur Verfügung steht.

In Yuki Gosei Kagaku Kyokai Shi 29 (1971) 12, 1129 (Chemical Abstract 76, 140 292 R) wird die Herstellung von Clevesäure-1,7 durch Sulfonierung von 1-Naphthol mit anschließender Hydrolyse zur 1-Naphthol-7-sulfonsäure und anschließender Bucherer-Reaktion beschrieben. Dieses Verfahren hat jedoch den Nachteil, daß es die Zwischenisolierung der 1-Naphthol-7-sulfonsäure verlangt und nur bei Verwendung wäßriger Salzsäure für die Hydrolyse zu guten Ausbeuten führt. Infolgedessen ist das Verfahren aufwendig und wirft erhebliche Korrosionsprobleme auf.

Ferner ist aus Donaldson, The Chemistry and Technology of Naphthalene Compounds, 1959, Seiten 198 und 209 bekannt, daß Clevesäure-1,7 bei der Hydrolyse von 1-Naphthylamin-2,7-disulfonsäure mit 80 %iger Schwefelsäure, oder bei der Hydrolyse von 1-Naphthylamin-2,4,7-trisulfonsäure in kochender 75 %iger Schwefelsäure, entsteht. Diese beiden Herstellungsweisen sind aber technisch deshalb uninteressant, weil für die Ausgangsverbindungen, die 1-Amino-naphthalin-2,4,7-trisulfonsäure und die 1-Aminonaphthalin-2,7-disulfonsäure, bislang noch keine wirtschaftlichen Herstellungsverfahren bekannt sind. Für die Herstellung der 1-Naphthylamin-2,4,7-trisulfonsäure wurden bislang folgende Verfahren vorgeschlagen:

1. Die Umsetzung von 1-Aminonaphthalin-4-sulfonsäure (Naphthionsäure) mit 3 bis 4 Teilen 40 %igem Oleum bei 120°C (siehe Friedländer I, Seite 331: DE-PS 22 545). Es wird aber bereits in der Beschreibung des patentierten Verfahrens darauf hingewiesen, daß die Trisulfonsäure nur in unbefriedigender Ausbeute erhalten wird, Napthionsäure durch Oleum in der Wärme oxidativ abgebaut wird. Außerdem wird in der Britischen Patentschrift No. 15 223 (1893) bei der Diskussion des in der Deutschen Patentschrift No. 22 545 beschriebenen Verfahrens darauf hingewiesen, daß bei der Umsetzung der Naphthionsäure mit Oleum mindestens 2 isomere Trisulfonsäuren, nämlich die 1-Aminonaphthalin-2,4,6-und die 1-Aminonaphthalin-2,4,7-trisulfonsäure entstehen.

2. Die Umsetzung von 1-Aminonaphthalin-7-sulfonsäure (Clevesäure-1,7) oder 1-Aminonaphthalin-4,7-disulfonsäure oder deren Salze mit Oleum bei Temperaturen von 50 bis 100°C (Britische Patentschrift No. 15 223 (1893). Dieses Verfahren ist technisch uninteressant, weil es als Ausgangsverbindungen Clevesäure-1,7 oder 1-Aminonaphthalin-4,7-disulfonsäure erfordert, die ihrerseits auch nur schwer und über viele Herstellungs-und Reinigungsstufen (zur Abtrennung der Isomeren) zugänglich sind. Das in Friedländer I, Seite 407 (=DE-PS 41 957) beschriebene Verfahren zur Herstellung von 1-Naphthylamin-4,7-disulfonsäure durch Sulfonierung von Naphthionsäure, ist für eine Durchführung in technischem Maßstab ungeeignet, weil 1-Naphthylamin-4,7-disulfonsäure im Gemenge mit der isomeren 1-Naphthylamin-4,6-disulfonsäure anfällt. Eine Trennung des anfallenden Disulfonsäuregemisches über die Calciumsalze wäre zwar möglich, ist aber technisch uninteressant. Für das Verfahren sind auch keine Ausbeuten angegeben.

Überraschenderweise wurde nun gefunden, daß man 1-Amino-naphthalin-2,4,7-trisulfonsäure in guten Ausbeuten und praktisch isomerenfrei aus technisch leicht zugänglichen Ausgangsverbindungen erhalten kann, wenn man 1-Nitronaphthalin durch Einwirkung von Hydrogensulfiten (d.h. nach Piria) reduzierend sulfoniert und das bei dieser Piria-Reaktion anfallende, im wesentlichen aus den Ammonium-und/oder Alkali-Sulfaminaten des 1-Aminonaphthalins, der 1-Naphthylamin-4-sulfonsäure (Naphthionsäure) und der 1-Naphthylamin-2,4-disulfonsäure bestehende Sulfaminat — Gemisch, das gegebenenfalls noch bei der Reaktion gebildete Ammonium-und/oder Alkali-Hydrogensulfate enthält, mit Schwefeltrioxid in Schwefelsäure sulfoniert.

Die Umsetzung von 1-Nitronaphthalin nach Piria mit Hydrogensulfiten ist an sich bekannt - (siehe z.B. R. Piria, Ann. 78 (1851), 31-68: J.Am.Chem.Soc. 53 (1931), 1432-1442 und 1443-1447: J.Am.Chem.Soc. 58 (1936), 225-228: J.Org.Chem. 13 (1948), 179). Da die Piria-Reaktion des 1-Nitronaphthalins nicht zu einer einheitlichen Verbindung, sondern zu Gemischen aus den Sulfaminaten des 1-Naphthylamins, der 1-Naphthylamin-4-sulfonsäure (Naphthionsäure) und der 1-Naphthylamin-2,4-disulfonsäure bzw., nach der Verseifung der Sulfaminate, zu Gemischen der entsprechenden freien Amine führt, aus der die einzelnen Amine nur mit Hilfe aufwendiger Trennverfahren isolierbar sind, hat die Piria-Reaktion des 1-Nitronaphthalins bislang keinerlei technische Bedeutung erlangt.

Erfindungsgemäß wurde gefunden, daß dieses bei der Piria-Reaktion des 1-Nitronaphthalins anfallende Sulfaminat-Gemisch ein äußerst günstiges Ausgangsmaterial für die Herstellung der 1-Aminonaphthalin-2,4,7-trisulfonsäure darstellt. Das Sulfaminat-Gemisch ist als solches einfach und aus billigen, technisch leicht zugänglichen Ausgangsverbindungen herstellbar und bei seiner Sulfonierung mit Schwefeltrioxid wird 1-Aminonaphthalin-2,4,7-trisulfonsäure in guten Ausbeuten praktisch isomerenfrei erhalten.

Überraschenderweise findet bei der Sulfonierung des Sulfaminat-Gemisches kein oxidativer Abbau statt und greift die Sulfonierung selektiv an den noch freien 2-, 4-und 7-Stellungen der Sulfaminate bzw. der aus ihnen während der Reaktion gebildeten Amine an. Erfindungsgemäß wurde gefunden, daß die im Sulfaminat-Gemisch enthaltenen Ammonium-und/oder Alkali-(hydrogen)sulfate und vor allem die Ammonium-und/oder Alkali-(hydrogen)sulfate, die sich bei der Sulfonierung des Sulfaminat-Gemisches bilden, eine selektive Sulfonierung der 2-, 4-und 7-Stellungen in den Sulfaminaten bewirken und den oxidativen Abbau verhindern.

Die Erfindung betrifft daher ein neues Verfahren zur Herstellung von 1-Aminonaphthalin-2,4,7-trisulfonsäure, das dadurch gekennzeichnet ist, daß man 1-Nitronaphthalin in an sich bekannter Weise nach Piria mit Hydrogensulfiten umsetzt, das bei dieser Piria-Reaktion anfallende Sulfaminat-Gemisch mit Schwefeltrioxid in Schwefelsäure sulfoniert und die entstandene 1-Aminonaphthalin-2,4,7-trisulfonsäure aus dem Sulfonierungsgemisch in an sich bekannter Weise isoliert.

Dieses neue Verfahren zur Herstellung der 1-Aminonaphthalin-2,4,7-trisulfonsäure eröffnet zugleich ein neues wirtschaftliches Verfahren zur Herstellung von Clevesäure-1,7, denn Clevesäure-1,7 ist aus der 1-Aminonaphthalin-2,4,7-trisulfonsäure durch Hydrolyse in guten Ausbeuten erhältlich.

Die Erfindung betrifft daher auch ein neues Verfahren zur Herstellung von Clevesäure-1,7, das dadurch gekennzeichnet ist, daß man 1-Nitronaphthalin in an sich bekannter Weise nach Piria mit Hydrogensulfiten umsetzt, das bei dieser Piria-Reaktion anfallende Sulfaminat-Gemisch mit Schwefeltrioxid sulfoniert und das Sulfonierungsgemisch oder die aus ihm isolierte 1-Aminonaphthalin-2,4,7-trisulfonsäure durch Erwärmen in wäßriger Schwefelsäure hydrolysiert.

Die erfindungsgemäße Sulfonierung des bei der Piria-Reaktion des 1-Nitronaphthalins anfallenden Sulfaminat-Gemisches wird bei Temperaturen von 10-150°C, vorzugsweise 30-120°C, vorgenommen.

Das für die Sulfonierung verwendete Schwefeltrioxid wird in Form von Oleum, vorzugsweise 20 bis 100 Gew. -%, besonders bevorzugt 40 bis 70 Gew. -% Schwefeltrioxid, enthaltendem Oleum eingesetzt. Das Oleum wird in einer solchen Menge angewendet, daß auf 1 Mol in die Piria-Reaktion eingesetztes 1-Nitronaphthalin 1,5 bis 10 Mol, vorzugsweise 2 bis 6 Mol, Schwefeltrioxid entfallen.

Die als Ausgangsmaterial für die erfindungsgemäße Sulfonierung zu verwendenden Sulfaminat-Gemische sind in an sich bekannter Weise erhältlich, beispielsweise, indem man 1-Nitronaphthalin mit wäßriger Ammoniumhydrogensulfit-Lösung, gegebenenfalls unter Zusatz von Ammoniak, oder mit wäßriger Alkalihydrogensulfit-Lösung, gegebenenfalls unter Zusatz von Alkali, solange auf Rückflußtemperatur erhitzt, bis eine klare Lösung entstanden ist. Diese wäßrige Lösung der Ammonium-und/oder Alakli-sulfaminate, sie besteht im wesentlichen aus einer Mischung der Sulfaminate des 1-Naphthylamins, der Naphthionsäure und der 1-Naphthylamin-2,4-disulfonsäure, und der bei der Reaktion gebildeten Ammonium-und/oder Alkali-hydrogensulfate wird vom Wasser befreit, beispielsweise durch Einengen am Rotationsverdampfer und anschließendes Trocknen des Rückstandes im Vakuum bei erhöhter Temperatur bis zu einem Rest Wassergehalt von 0 bis 10 Gew.-%.

Ein besonders günstiges Ausgangsmaterial für die erfingungsgemäße Sulfonierung wird beispielsweise erhalten, wenn man 1 Mol 1-Nitronaphthalin, suspendiert in 1 bis 2 l Wasser, mit 3 bis 5 Mol Ammoniumhydrogensulfit, vorzugsweise in Form einer 40 bis 50 gew.-%igen wäßrigen Lösung, und 1,5 bis 3 Mol Ammoniak, vorzugsweise in Form einer etwa 25 gew.-%igen wäßrigen Lösung solange auf 100 bis 105°C erhitzt, bis eine klare Lösung entstanden ist. Während der Umsetzung fällt der pH-Wert der Lösung von 6 bis 6,5 auf 5 bis 5,5. Nach dem Eindampfen der Reaktionslösung im Rotationsverdampfer und Trocknen des Rückstandes im Vakuum wird ein Produkt folgender Zusammensetzung erhalten:

9,1 Gew.-% $1\text{-NH-SO}_3\text{NH}_4\text{-C}_{10}\text{H}_6\text{-4-SO}_3\text{NH}_4$

49,3 Gew.-% $1\text{-NH-SO}_3\text{NH}_4\text{-C}_{10}\text{H}_5\text{-2,4-(SO}_3\text{NH}_4)_2$

3,0 Gew.-% $1\text{-NH-SO}_3\text{NH}_4\text{-C}_{10}\text{H}_6\text{-2-SO}_3\text{NH}_4$

0,7 Gew.-% $1\text{-NH-SO}_3\text{NH}_4\text{-C}_{10}\text{H}_5\text{-4,7-(SO}_3\text{NH}_4)_2$

1,8 Gew.-% Wasser

36,1 Gew.-% Ammoniumhydrogensulfat + gegebenenfalls geringe Mengen an Ammoniumhydrogensulfit und organischen Nebenprodukten unbekannter Struktur.

Die Zusammensetzung des Sulfaminat-Gemisches wurde mittels Hochdruck-Flüssigkeitschromatographie (HPLC) nach Spaltung der Sulfaminate mit Mineralsäure bestimmt.

Falls die als Ausgangsmaterial für die erfindungsgemäße Sulfonierung einzusetzenden Sulfaminat-Gemische noch Wasser enthalten, ist es ratsam, für die Sulfonierung außer der für die Sulfonierung erforderlichen Schwefeltrioxid(Oleum)-Menge noch zusätzlich Schwefeltrioxid (Oleum) zum Binden des Wassers zuzusetzen.

Die erfindungsgemäße Sulfonierung des Sulfaminat-Gemisches kann auf verschiedene Weise vorgenommen werden:

Beispielsweise kann man zunächst das Sulfaminat-Gemisch in wasserfreier Schwefelsäure suspendieren, die Suspension langsam mit der vorgesehenen Oleum-Menge versetzen und während der Oleum-Zugabe die Temperatur des Reaktionsgemisches stufenlos oder stufenweise von 10°C auf 150°C, vorzugsweise von 30°C auf 120°C, erhöhen und die Sulfonierung bei 80 bis 150°C, vorzugsweise 90 bis 120°C, zu Ende führen.

Um die Rührfähigkeit der Reaktionsgemische zu verbessern, hat es sich jedoch als vorteilhafter erwiesen, wasserfreie $H_2SO_4$ vorzulegen und in diese Vorlage bei Temperaturen von 10 bis 150°C, vorzugsweise 30 bis 120°C, gleichzeitig das getrocknete Sulfaminat-Gemisch und die erforderliche Oleum-Menge einzudosieren und die Reaktion bei 80 bis 150°C, vorzugsweise 90 bis 120°C, zu Ende zu führen.

Das nach Abschluß der Sulfonierung vorliegende Sulfonierungsgemisch ist eine Lösung oder Suspension von 1-Aminonaphthalin-2,4,7-trisulfonsäure in Schwefelsäure: sie enthält nur geringe Anteile an isomerer 1-Aminonaphthalin-2,4,6-trisulfonsäure. Die 1-Aminonaphthalin-2,4,7-trisulfonsäure kann aus dem Sulfonierungsgemisch durch Verdünnen mit Wasser isoliert werden. Man kann das Sulfonierungsgemisch jedoch auch unmittelbar zur Herstellung der Clevesäure-1,7 verwenden.

Die Hydrolyse der 1-Aminonaphthalin-2,4,7-trisulfonsäure zur Clevesäure-1,7 wird in 60 bis 80 %iger, vorzugsweise 60 bis 75 %iger wäßriger Schwefelsäure bei Temperaturen von 140 bis 170°C, vorzugsweise von 145 bis 165°C, vorgenommen. Die Hydrolysedauer beträgt etwa 0,5 bis 10 Stunden.

Die Clevesäure-1,7, läßt sich aus dem Hydrolysegemisch durch Verdünnen auf eine 30 bis 60 %ige Schwefelsäure (Gewichtsprozent) ausfällen. Die ausgefallene Säure wird abgesaugt, mit Wasser gewaschen und getrocknet. Sie ist frei von Clevesäure-1,6.

Die Clevesäure-1,7 fällt als freie Säure an: sie kann gewünschtenfalls in bekannter Weise, z.B. durch Neutralisation ihrer wäßrigen Aufschlämmung mit entsprechenden Basen, in die gewünschten Salze überführt werden.

Für den -bevorzugten -Fall, daß man das bei der Sulfonierung des Sulfaminat-Gemisches anfallende Sulfonierungs gemisch unmittelbar hydrolysiert, wird das Sulfonierungsgemisch einfach mit so viel Wasser vermischt, daß eine 60 bis 80 %ige Schwefelsäure entsteht. Das Vermischen des Sulfonierungsgemisches mit dem Wasser wird vorteilhaft in der Weise vorgenommen, daß man das Sulfonierungsgemisch in das Wasser einträgt und dabei die Temperatur möglichst nahe an der Siedetemperatur der Hydrolysemischung hält. Anschließend wird das Gemisch mehrere Stunden bei 140 bis 170°C gerührt, und nach Beendigung der Hydrolyse mit weiterem Wasser zum Ausfällen der Clevesäure-1,7 versetzt.

Für die Isolierung der Clevesäure-1,7 kann es auch zweckmäßig sein, nach Beendigung der Hydrolyse die Schwefelsäure durch, Zugabe von Calciumcarbonat und/oder Calciumoxid/hydroxid als Gips abzutrennen und die Clevesäure-1,7 nach dem Abfiltrieren des Gipses aus dem Filtrat durch Ansäuern mit einer Mineralsäure, vorzugsweise Salzsäure, in einer sehr gut filtrierbaren Form auszufällen.

Beispiel 1

a) Herstellung des Sulfaminat-Gemisches:

In einem mit Rührer, Rückflußkühler, Glaselektrode und Innenthermometer versehenen 2 1-Vierhalskolben wird eine Mischung aus 86,5 g (0,5 Mol) 1-Nitronaphthalin, 70 g (1,0 Mol) 25 gew.-%ige NH$_3$-Lösung, 427 g (2,0 Mol) 46,4 gew.-%ige wäßrige NH$_4$HSO$_3$-Lösung und 700 g Wasser solange auf Rückflußtemperatur (etwa 101°C) erhitzt, bis das 1-Nitronaphthalin vollständig in Lösung gegangen ist (Erhitzungsdauer: etwa 5 Stunden). Während des Erhitzens sinkt der pH-Wert der Reaktionsmischung von 6,3 auf 5,2.

Anschließend wird die Reaktionslösung im Rotationsverdampfer zur Trockne eingeengt und im Vakuum bei 100°C bis zur Gewichtskonstanz getrocknet. Es werden 276 g Product folgender Zusammensetzung erhalten:

9,1 Gew.-% 1-NH-SO$_3$NH$_4$-C$_{10}$H$_6$-4-SO$_3$NH$_4$ MG 337

49,3 Gew.-% 1-NH-SO$_3$NH$_4$-C$_{10}$H$_5$-2,4-(SO$_3$NH$_4$)$_2$ MG 434

3,0 Gew.-% 1-NH-SO$_3$NH$_4$-C$_{10}$H$_6$-2-SO$_3$NH$_4$ MG 337

0,7 Gew.-% 1-NH-SO$_3$NH$_4$-C$_{10}$H$_5$-4,7-(SO$_3$NH$_4$)$_2$ MG 434

1,8 Gew.-% Wasser

36,1 Gew.-% Ammoniumhydrogensulfat + gegebenenfalls geringe Mengen an Ammoniumhydrogensulfit und organischen Nebenprodukten unbekannter Struktur.

Die Zusammensetzung des Produktes wurde durch HPLC nach saurer Hydrolyse der Sulfaminate mittels einer Mineralsäure bestimmt.

b) Sulfonierung des Sulfaminat-Gemisches:

In einem mit Rührer, Innenthermometer, Kühler, Dosier-Tropftrichter und Feststoff-Dosierer ausgerüsteten Sulfierkolben werden 196 g (2,0 Mol) 100 gew.-%ige H$_2$SO$_4$ vorgelegt und auf 95 bis 100°C erwärmt. Nach Spülen der Apparatur mit trockenem Stickstoff werden im Verlaufe von 2 h bei 95 bis 100°C gleichzeitig 200 g 65 %iges Oleum (1,6 Mol SO$_3$, davon 0,22 Mol zum Binden des im Sulfaminat-Gemisch enthaltenden Wassers) eingetropft und 221 g Sulfaminat-Gemisch (≙ 0,4 Mol 1-Nitronaphthalin) eindosiert.

Die Reaktionsmischung wird 3 h bei 95 bis 100°C nachgerührt. Mittels HPLC wurde folgende Zusammensetzung des Sulfonierungsgemisches ermittelt:

17,8 Gew.-% 1-Naphthylamin-2,4,7-trisulfonsäure MG 383

0,2 Gew.-% 1-Naphthylamin-2,4,6-trisulfonsäure MG 383

1,2 Gew.-% 1-Naphthylamin-2,4-disulfonsäure MG 303

0,2 Gew.-% 1-Naphthylamin-4,6-disulfonsäure MG 303

0,1 Gew.-% 1-Naphthylamin-4,7-disulfonsäure MG 303

Damit beträgt die Ausbeute an 1-Naphthylamin-2,4,7-trisulfonsäure 72 % d.Th., bezogen auf eingesetztes 1-Nitronaphthalin.

c) Saure Hydrolyse des Sulfonierungsgemisches und Isolierung der Clevesäure-1,7:

In das Sulfonierungsgemisch läßt man bei 100°C im Verlaufe von etwa 1 h 170 g Wasser einlaufen; dabei steigt die Temperatur auf etwa 130°C an. Die Reaktionsmischung wird bis zu ihrem Siedepunkt (etwa 155°C) erwärmt und etwa 2 h auf dieser Temperatur gehalten.

Die Analyse des Hydrolysegemisches mittels HPLC ergab folgende Zusammensetzung:

6,60 Gew.-% 1-Naphthylamin-7-sulfonsäure MG 223

0,07 Gew.-% 1-Naphthylamin-6-sulfonsäure MG 223

0,23 Gew.-% 1-Naphthylamin-4,7-disulfonsäure MG 303

0,21 Gew.-% 1-Naphthylamin-4,6-disulfonsäure MG 303

0,23 Gew.-% 1-Naphthylamin-2,7-disulfonsäure MG 303.

Damit beträgt die Ausbeute an 1-Naphthylamin-7-sulfonsäure ca. 58 % d.Th., bezogen auf eingesetztes 1-Nitro-naphthalin.

Zur Isolierung der Clevesäure-1,7 wird die Reaktionsmischung mit etwa 400 g Wasser verdünnt, die Schwefelsäure durch Zugabe von etwa 850 g 60 gew.-%iger wäßriger $CaCO_3$-Suspension in $CaSO_4$ überführt, der Gips abfiltriert und mit Wasser gewaschen. Die vereinigten Filtrate werden bei 80°C mit etwa 190 g 30 gew.-%iger HCl bis zu einem pH-Wert von etwa 1 angesäuert. Die auf diese Weise erhaltene Suspension wird im Verlaufe mehrerer Stunden auf etwa 20°C abgekühlt; die ausgefallene Clevesäure-1,7 wird abfiltriert und mit kaltem Wasser gewaschen. Es werden 58 g Clevesäure-1,7 (feucht) erhalten mit folgender mittels HPLC bestimmter Zusammensetzung:

78,1 Gew.-% 1-Naphthylamin-7-sulfonsäure MG 223

0,3 Gew.-% 1-Naphthylamin-6-sulfonsäure MG 223

0,5 Gew.-% 1-Naphthylamin-4,7-disulfonsäure MG 303.

Damit beträgt die Ausbeute an isolierter Clevesäure-1,7 ca. 51 % d.Th., bezogen auf eingesetztes 1-Nitronaphthalin.

Beispiel 2

a) Unter den in Beispiel 1 (a) beschriebenen Bedingungen werden aus 173 g (1,0 Mol) 1-Nitronaphthalin, 140 g (2,0 Mol) 25 gew.-%iger wäßriger $NH_3$-Lösung, 778 g (4,0 Mol) 51 gew.-%iger wäßriger $NH_4HSO_3$-Lösung und 1500 g $H_2O$ 583 g Sulfaminat-Gemisch (getrocknetes Produkt) erhalten.

b) In der in Beispiel 1 beschriebenen Sulfier-Apparatur werden 235 g (2,4 Mol) 100 gew.%ige $H_2SO_4$ vorgelegt. Bei 10 bis 15°C werden gleichzeitig 222 g 65 %iges Oleum (1,8 Mol $SO_3$) eingetropft und 233 g (≙ 0,4 Mol 1-Nitronaphthalin) Sulfaminat-Gemisch eindosiert. Die Reaktionsmischung wird auf 95°C erwärmt, 2 h bei 95°C gerührt, langsam mit 160 g $H_2O$ versetzt, wobei die Temperatur auf 155°C ansteigt, 90 Min. bei 155°C gerührt, mit 400 g $H_2O$ versetzt und auf 50°C abgekühlt. die ausgefallene Clevesäure-1,7 wird abfiltriert und mehrfach mit kaltem Wasser gewaschen. Es werden 86 g Clevesäure-1,7 (feucht) erhalten mit folgender, mittels HPLC bestimmter Zusammensetzung:

51,0 Gew.-% 1-Naphthylamin-7-sulfonsäure MG 223

0,05 Gew.-% 1-Naphthylamin-4,7-disulfonsäure MG 303

1,2 Gew.-% 1-Naphthylamin-2,7-disulfonsäure MG 303.

Damit beträgt die Ausbeute an isolierter Clevesäure-1,7 49 % der Theorie, bezogen auf eingesetztes 1-Nitronaphthalin.

Beispiel 3

a) In der Beispiel 1 (a) beschriebenen Apparatur werden 86,5 g (0,5 Mol) 1-Nitronaphthalin, 20 g (0,5 Mol) NaOH, 190 g $Na_2S_2O_5$ (1,0 Mol ≙ 2,0 Mol $NaHSO_3$) und 1000 g $H_2O$ auf Rückflußtemperatur erhitzt bis eine klare Lösung entstanden ist. Nach dem Trocknen werden 293 g Sulfaminat-Gemisch folgender Zusammensetzung erhalten (ermittelt durch HPLC nach saurer Hydrolyse mit Mineralsäuren):

8,1 Gew.-% 1-$NH$-$SO_3Na$-$C_{10}H_6$-4-$SO_3Na$ MG 347

47,9 Gew.-% 1-$NH$-$SO_3Na$-$C_{10}H_5$-2,4-$(SO_3Na)_2$ MG 449

3,4 Gew.-% 1-$NH$-$SO_3Na$-$C_{10}H_7$ MG 245

2,0 Gew.-% $H_2O$

38,6 Gew.-% $NaHSO_4$ + organische Nebenprodukte unbekannter Struktur.

b) In der in Beispiel 1 (b) beschriebenen Sulfier-Apparatur werden 196 g (2,0 Mol) 100 gew.-%ige $H_2SO_4$ vorgelegt. Bei 30 bis 40°C werden gleichzeitig im Verlaufe von 2 h 260 g 65 gew.-%iges Oleum (2,1 Mol $SO_3$, davon 0,26 Mol für ein Binden des Wassers im Sulfaminat-Gemisch) eingetropft und 234,5 g (≙ 0,4 Mol 1-Nitronaphthalin) Sulfaminat-Gemisch eindosiert.

Die Reaktionsmischung wird 4 h bei 90°C nachgerührt. Die Analyse des Sulfonierungsgemisches mittels HPLC ergab folgende Zusammensetzung:

16,9 Gew.-% 1-Naphthylamin-2,4,7-trisulfonsäure MG 383

0,5 Gew.-% 1-Naphthylamin-2,4,6-trisulfonsäure MG 383

0,22 Gew.-% 1-Naphthylamin-2,4-disulfonsäure MG 303

0,14 Gew.-% 1-Naphthylamin-4,7-disulfonsäure MG 303

0,07 Gew.-% 1-Naphthylamin-4,6-disulfonsäure MG 303.

Damit beträgt die Ausbeute an 1-Naphthylamin-2,4,7-trisulfonsäure 76 % d.Th., bezogen auf eingesetztes 1-Nitronaphthalin.

c) Das Sulfonierungsgemisch wird im Verlaufe von etwa 1 h in 205 g $H_2O$ von 80°C eingetragen, wobei die Temperatur auf 155°c ansteigt. Die Reaktionsmischung wird 5 h auf 155°C gehalten, mit 245 g $H_2O$ verdünnt, auf 50°C abgekühlt und etwa 30 Min. bei 50°C gerührt. Die ausgefallene Clevesäure-1,7 wird abfiltriert, mehrfach mit kaltem Wasser säurefrei gewaschen und im Vakuum bei 80°C getrocknet. Es werden 43,5 g Clevesäure-1,7 (trocken) folgender Zusammensetzung erhalten:

96,1 Gew.-% 1-Naphthylamin-7-sulfonsäure MG 223

<0,05 Gew.-% 1-Naphthylamin-6-sulfonsäure MG 223

1,0 Gew.-% 1-Naphthylamin-2-sulfonsäure MG 223

0,1 Gew.-% 1-Naphthylamin-4,7-disulfonsäure MG 303

<0,05 Gew.-% 1-Naphthylamin-2,4-disulfonsäure MG 303

<0,05 Gew.-% 1-Naphthylamin-2,7-disulfonsäure MG 303

<0,05 Gew.-% 1-Naphthylamin-4,6-disulfonsäure MG 303.

Damit beträgt die Ausbeute an isolierter Clevesäure-1,7 47 % d.Th., bezogen auf eingesetztes 1-Nitronaphthalin.

## Ansprüche

1. Verfahren zur Herstellung von 1-Aminonaphthalin-2,4,7-trisulfonsäure, dadurch gekennzeichnet, daß man 1-Nitronaphthalin in an sich bekannter Weise nach Piria mit Hydrogensulfiten umsetzt, das bei dieser Piria-Reaktion anfallende Sulfaminat-Gemisch mit Schwefeltrioxid in Schwefelsäure sulfoniert und die 1-Aminonaphthalin-2,4,7-trisulfonsäure aus dem Sulfonierungsgemisch in an sich bekannter Weise isoliert.

2. Verfahren nach Anspruch 1, dadurch gekennzeichnet, daß man das Schwefeltrioxid in Form von Oleum einsetzt.

3. Verfahren nach Anspruch 2, dadurch gekennzeichnet, daß man das Schwefeltrioxid in Form von 20 bis 100 gew.-%igem Oleum einsetzt.

4. Verfahren nach Ansprüchen 1 bis 3, dadurch gekennzeichnet, daß man die Menge an Schwefeltrioxid so bemißt, daß auf 1 Mol in die Piria-Reaktion eingesetztes 1-Nitronaphthalin 1,5 bis 10 Mol Schwefeltrioxid entfallen.

5. Verfahren nach Ansprüchen 1 bis 4, dadurch gekennzeichnet, daß man als Sulfaminat-Gemische solche Gemische einsetzt, wie sie beim Einengen solcher wäßrigen Reaktionslösungen anfallen, die beim Erhitzen von 1 Mol 1-Nitronaphthalin, suspendiert in Wasser, mit der wäßrigen Lösung von 3 bis 5 Mol Ammonium-oder Alkali-hydrogensulfit und 1,5 bis 3 Mol Ammoniak oder wäßriger Alkalilauge auf 100 bis 105°C erhalten werden.

6. Verfahren zur Herstellung von Clevesäure-1,7, dadurch gekennzeichnet, daß man 1-Nitronaphthalin in an sich bekannter Weise nach Piria mit Hydrogensulfiten umsetzt, das bei dieser Piria-Reaktion anfallende Sulfaminat-Gemisch mit Schwefeltrioxid in Schwefelsäure sulfoniert und das Sulfonierungsgemisch nach Einstellung der gewünschten Schwefelsäurekonzentration hydrolysiert.

7. Verfahren gemäß Anspruch 6, dadurch gekennzeichnet, daß man die Hydrolyse in 60 bis 80 %iger Schwefelsäure vornimmt.